# FASCICULE DE BREVET EUROPEEN

(11) **EP 1 545 698 B1**
(45) Date de publication et mention de la délivrance du brevet: **09.04.2008**
(21) Numéro de dépôt: 03798007.5
(22) Date de dépôt: 19.08.2003
(51) Int. Cl.: A61N 1/32, A61N 1/04

(54) **DISPOSITIF DE TRAITEMENT CORPOREL PAR ELECTROSTIMULATION OU IONOPHORESE**
VORRICHTUNG ZUR BEHANDLUNG DES KÖRPERS MIT ELEKTROSTIMULATION ODER IONTOPHORESE
DEVICE FOR BODY TREATMENT BY ELECTROSTIMULATION OR IONTOPHORESIS

(30) Priorité: 30.09.2002 EP 02447186
(43) Date de publication de la demande: 29.06.2005
(73) Titulaire: FACO S.A., B-4020 Wandre (BE)
(72) Inventeur: JULEMONT, Pierre, B-4601 Argenteau (BE); SMAL, Olivier, B-4053 Embourg (BE)
(74) Mandataire: pronovem
(86) Numéro de dépôt international: PCT/BE2003/000138
(87) Numéro de publication internationale: WO 2004/028625

(56) Documents cités:
- EP-A- 0 906 767
- US-A- 5 498 235
- US-A- 5 991 655
- US-A- 6 157 858

## Description

### Objet de l'invention

La présente invention se rapporte à un appareil de traitement corporel par voie électrique, en particulier d'électrostimulation convenant notamment pour l'amélioration de la performance musculaire, mais également destiné à des finalités de massage, de détente, d'amélioration de la silhouette. Ledit dispositif peut également convenir pour l'ionophorèse pour des finalités d'épilation.

### Etat de la technique

En vue du traitement corporel pour les finalités prémentionnées, on utilise un certain nombre de dispositifs de traitement corporel par voie électrique consistant à appliquer des électrodes sur le corps.

Les électrostimulateurs sont généralement destinés à créer des impulsions électriques agissant sur les muscles afin de provoquer une contraction musculaire dans des buts thérapeutiques ou pour augmenter le développement de la musculature. Les impulsions électriques sont généralement programmées à des fréquences pouvant être de l'ordre de 40 à 60 Hz selon des séquences prédéfinies en fonction des effets désirés (entraînement, musculation, récupération, ...).

De nombreux appareils d'électrostimulation, qu'ils soient à usage strictement médical ou encore destinés à un usage en kinésithérapie ou à un usage individuel sont connus.

Les appareils d'ionophorèse sont plus spécifiquement utilisés à des finalités d'épilation et reposent sur l'utilisation d'électrodes alimentées par un courant continu de faible intensité.

La plupart des dispositifs présentent l'inconvénient d'être constitués par un certain nombre d'électrodes à fixation mécanique, par exemple par une sangle, ou adhésives, qui sont reliées à un boîtier de commande à l'aide de fils. Le boîtier de commande réglable peut être alimenté par des piles ou être raccordé au réseau électrique et comporte bien entendu dans ce cas un transformateur produisant un courant aux tensions et intensités adaptés.

Des éléments les plus simples qui ont été proposés, sont autonomes en ce sens qu'ils comportent une alimentation électrique sous forme de batteries ou de piles et un dispositif électronique créateur d'ondes.

Généralement, l'intensité du courant appliqué aux électrodes est programmable, individuellement ou par groupes d'électrodes et un dispositif individuel comporte deux ou plus de deux électrodes reliées à un boîtier individuel.

Il est souhaitable cependant que la distance entre les électrodes soit modulable tout en évitant le recours à des fils et autres moyens compliqués se révélant rapidement encombrants à l'usage et pour le rangement. L'utilisateur souhaite aussi disposer de moyens simples et peu encombrants pour la fixation des électrodes.

Gross dans le brevet US-A-6,157,858 décrit un dispositif pour l'administration de produits pharmaceutiques avec un contrôleur électronique se trouvant dans un boîtier collé sur la peau. Le brevet US-A-6 157 858 divulgue un dispositif selon le préambule de la revendication 1.

Dans le cadre de la présente invention, le boîtier électronique n'est pas collé sur la peau mais est maintenu en place par la rigidité des glissières, ce qui évite un point de fixation qui n'est pas absolument nécessaire et ce qui donne pour un même nombre d'électrodes, une plus grande liberté de mouvement au porteur de ces électrodes.

### Buts de l'invention

La présente invention vise à fournir un dispositif du type mentionné conférant à ses utilisateurs une autonomie complète de mouvement et des possibilités élargies d'ajustage, de programmation et de synchronisation des électrodes.

### Eléments caractéristiques de l'invention

Le dispositif selon l'invention est défini par la revendication 1. Les revendications dépendantes définissent des modes de réalisation de l'invention.

Le dispositif selon l'invention est constitué par un boîtier relié à au moins deux électrodes, caractérisé en ce que l'élément de liaison entre le boîtier et au moins une électrode est constitué d'une bande de raccordement formée d'une matière souple, mais de préférence suffisamment rigide dans le sens transversal, pourvue d'un conducteur capable de se rétracter ou de coulisser dans le boîtier tout en maintenant le contact électrique dans ledit boîtier, de manière à pouvoir faire varier la distance entre les électrodes.

De préférence, le boîtier est équipé d'au moins deux bandes de raccordement chacune pourvues d'une électrode. On peut envisager cependant que le boîtier lui-même comporte une ou plusieurs électrodes qui lui sont propres.

Avantageusement, les électrodes sont montées individuellement, de préférence de manière amovible, sur l'extrémité libre de la bande de raccordement de manière pivotante en assurant un contact électrique et de manière orientable pour pouvoir en plus adapter la position angulaire de l'électrode par rapport à l'axe longitudinale de ladite bande.

La bande de raccordement est suffisamment souple pour pouvoir s'adapter à une forme quelconque arrondie du corps. Du fait de sa largeur, la bande, si elle peut s'adapter à la forme du corps, est cependant suffisamment rigide dans le sens transversal, c'est-à-dire qu'elle n'est que très faiblement déformable dans ce sens transversal.

Selon une caractéristique supplémentaire et particulièrement intéressante de l'invention, plusieurs dispositifs du type décrit, c'est-à-dire comportant un boîtier central de commande et au moins deux électrodes, peuvent être utilisés simultanément.

On constate cependant que ceci peut entraîner certains inconvénients ou un inconfort pour l'utilisateur, en particulier dans le cas des éléctrostimulateurs, en cas d'absence de « synchronisation » des ondes produites.

Selon une caractéristique supplémentaire de l'invention, on a en conséquence prévu que chacun des boîtiers, qui peuvent être équipés d'un dispositif individuel de commande manuelle de l'intensité du courant, peut aussi être télécommandé par l'utilisateur sans contact physique à l'aide d'un boîtier-maître, par exemple par infrarouge, radio, ou encore en utilisant le corps et en particulier la peau comme conducteur, ou tout autre moyen.

Ceci contribue largement au confort de l'utilisateur, pour éviter les inconvénients précités pouvant résulter d'un réglage défectueux de l'appareil. Le boîtier-maître comporte dans ce cas les éléments électroniques nécessaires pour empêcher un défaut notamment de synchronisation qui pourrait être gênant pour l'utilisateur.

### Brève description des figures

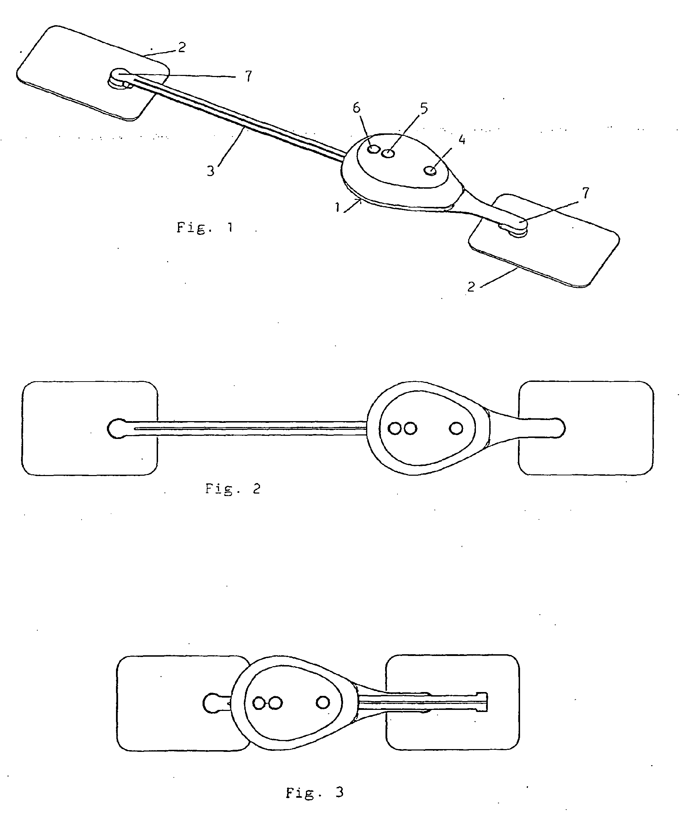

La figure 1 représente une vue en perspective en position déployée d'une des deux électrodes de la présente invention.

La figure 2 représente une vue en plan correspondant à la figure 1.

La figure 3 représente une vue correspondant à la figure 2 mais en position rétractée des deux électrodes.

Les figures 4 et 5 représentent respectivement des vues schématiques de plusieurs dispositifs selon l'invention commandés par un boîtier-maître. Dans la figure 5, le boîtier-maître est dépourvu lui-même d'électrodes, et dans la figure 6, il est équipé d'électrodes qui lui sont propres.

Des indices de référence identiques sont utilisés dans les différentes figures pour des éléments identiques ou fonctionnellement similaires.

### Description détaillée de l'invention

L'invention sera décrite essentiellement en référence à un dispositif d'électrostimulation à titre d'illustration.

Le dispositif d'électrostimulation selon la présente invention présente l'avantage d'offrir une autonomie complète de mouvement. Ceci permet notamment à l'utilisateur de porter un tel dispositif sous les vêtements sans interrompre ses activités.

Ce dispositif comporte un boîtier de commande individuel 1 muni d'un commutateur 4 à fonction « ON/OFF » destiné à la mise en marche et éventuellement au choix des séquences d'impulsions à fonction « MODE » ainsi que de commutateurs 5 et 6 permettant d'augmenter ou de diminuer la puissance des impulsions choisies.

Les électrodes sont constituées par des surfaces conductrices adhésives 2 autocollantes, c'est-à-dire réutilisables, et, de préférence, dont le pouvoir collant est réactivable par exemple par mouillage ou toute autre technique. Chaque électrode est reliée au boîtier de commande 1 par une bande conductrice 3 pouvant coulisser individuellement dans le boîtier 1 et permettant un ajustement de la distance entre les électrodes individuelles 2, tout en préservant le contact électrique.

Les bandes conductrices sont connectées aux électrodes par l'intermédiaire d'un point de pivotement 7 décalé par rapport au centre de l'électrode qui permet le déplacement ou le re-positionnement d'une des deux électrodes reliées au boîtier sans nécessairement déplacer l'autre.

Ces bandes conductrices sont relativement plates et sont donc déformables (souples), ce qui leur permet de s'adapter parfaitement aux formes du corps tout en présentant une rigidité dans le sens longitudinal.

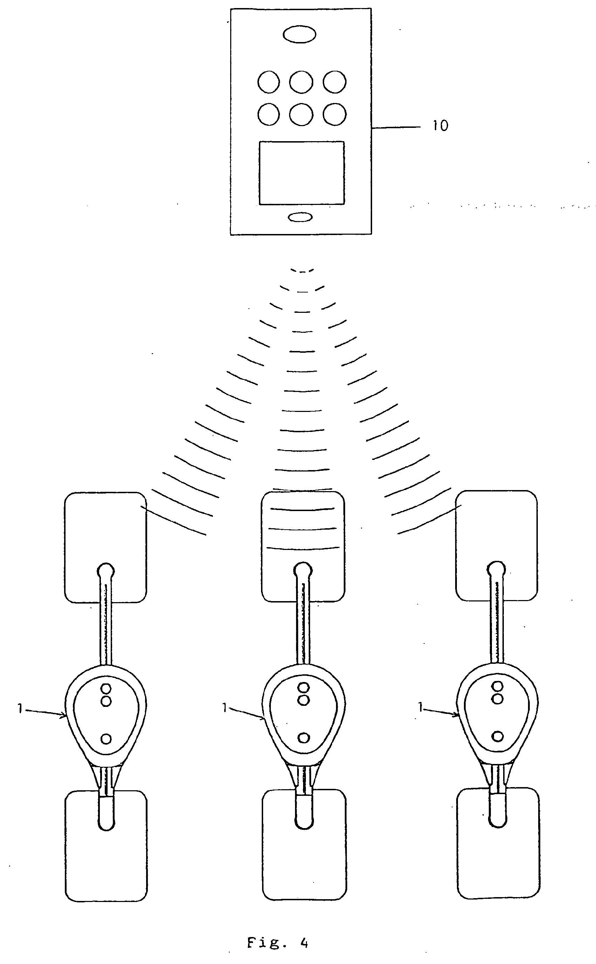

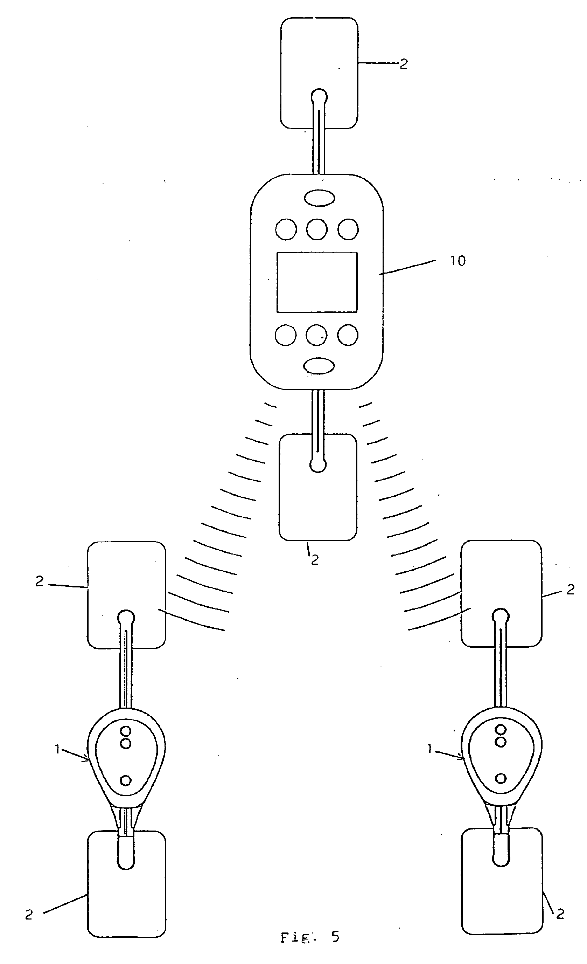

Différents boîtiers de commande 1 du type indiqué peuvent eux-mêmes être commandés par un boîtier-maître 10 (voir représentation schématisée aux figures 4 et 5) permettant ainsi une synchronisation optimale entre les unités individuelles pour éviter les sensations désagréables d'une désynchronisation non souhaitée des impulsions.

Avantageusement, le boîtier-maître 10 agit comme une télécommande par infrarouge, une radiocommande ou tout autre moyen pouvant convenir, pour les boîtiers de commande 1 individuels. On peut également lorsque le boîtier-maître est appliqué sur le corps, utiliser la peau elle-même comme conducteur. En particulier, selon la forme d'exécution de la figure 5, en plus de transmettre les messages de commande depuis le boîtier-maître 10 vers des boîtiers individuels, ce boîtier-maître comporte deux électrodes 2.

Il est particulièrement simple d'utiliser un dispositif de ce type pour des exercices musculaires ou d'électrostimulation de tous types avec une grande variété d'utilisation ou zones d'application (abdominaux, pectoraux, nuque, cuisse, etc.).

Les dispositifs décrits peuvent également être utilisés en ionophorèse par courant continu de faible voltage dans des buts d'épilation.

## Revendications

1. Dispositif de traitement corporel électrique comprenant un boîtier de commande individuel (1), au moins deux électrodes (2) reliées au boîtier, un élément de liaison (3) reliant le boîtier (1) à au moins une des électrodes (2), l'élément de liaison étant constitué d'une bande de raccordement formé d'une matière souple pourvue d'un conducteur, **caractérisé en ce que** l'élément de liaison (3) est capable de se rétracter ou de coulisser dans le boîtier (1) tout en maintenant le contact électrique dans ledit boîtier (1), de manière à pouvoir faire varier la distance entre les électrodes (2).

2. Dispositif selon la revendication 1 **caractérisé en ce que** chaque électrode (2) est montée sur l'extrémité libre de ladite bande de manière pivotante (7) et de manière amovible.

3. Dispositif selon la revendication 1 ou 2, **caractérisé en ce que** ladite bande de raccordement est suffisamment souple pour pouvoir s'adapter à une forme quelconque arrondie du corps tout en étant suffisamment rigide dans le sens transversal pour maintenir sa position sur le corps.

4. Dispositif selon l'une quelconque des revendications 1 à 3, **caractérisé en ce que** le boîtier de commande individuel comprend un commutateur de mise en marche et de choix des séquences d'impulsions (4) et des commutateurs (5 et 6) permettant l'augmentation et la diminution des impulsions.

5. Dispositif selon l'une quelconque des revendications 1 à 4, **caractérisé en ce que** plusieurs boîtiers de commande individuels (1) sont commandés par l'utilisateur à l'aide d'un boîtier-maître (10).

6. Dispositif selon la revendication 5, **caractérisé en ce que** le boîtier-maître (10) comporte des éléments électroniques nécessaires pour le réglage des programmes de séquences d'impulsions et/ou le réglage de l'intensité de façon individuelle, tout en empêchant un défaut de synchronisation des boîtiers de commande individuels (1).

7. Dispositif selon l'une quelconque des revendications 5 ou 6 **caractérisé en ce que** le boîtier-maître (10) agit par télécommande sur les boîtiers de commande individuels (1).

8. Dispositif selon l'une quelconque des revendications 5 ou 6 **caractérisé en ce que** le boîter-maître (10) est appliqué sur la peau et envoie les signaux de commande aux boîtiers de commande individuels (1) par l'intermédiaire de la peau.

## Claims

1. Device for the electric treatment of the body comprising an individual control box (1), at least two electrodes (2) connected to the box, a linking element (3) connecting the box (1) to at least one of the electrodes (2), the linking element comprising a connecting strip made of a flexible material with a conductor, **characterised in that** the linking element (3) can retract or slide in the box (1) whilst maintaining the electric contact in said box (1) in such a way as to vary the distance between the electrodes (2).

2. Device according to Claim 1, **characterised in that** each electrode (2) is pivotably (7) and detachably attached to the free end of said strip.

3. Device according to Claim 1 or 2, **characterised in that** said connecting strip is flexible enough to adapt to any curved shape of the body whilst being rigid enough in the transversal direction to maintain its position on the body.

4. Device according to any one of Claims 1 to 3, **characterised in that** the individual control box comprises a switch (4) for starting and selecting the sequences of pulses and switches (5 and 6) allowing to increase or decrease the pulses.

5. Device according to any one of Claims 1 to 4, **characterised in that** several individual control boxes (1) are controlled by the user by means of a master box (10).

6. Device according to Claim 5, **characterised in that** the master box (10) comprises the necessary electronic elements to individually adjust the programs for the pulse sequences and/or adjust the intensity, whilst preventing synchronisation faults of the individual control boxes (1).

7. Device according to any one of Claim 5 or 6, **characterised in that** the master box (10) acts by remote control on the individual control boxes (1).

8. Device according to any one of Claim 5 or 6, **characterised in that** the master box (10) is applied to the skin and sends the control signals to the individual control boxes (1) through the skin.

## Patentansprüche

1. Vorrichtung zur elektrischen Körperbehandlung, die aus einer Einzelsteuerung (1) besteht, mindestens zwei Elektroden (2), die mit der Steuerung verbunden sind, und einem Verbindungselement (3), das die Steuerung (1) mit mindestens einer der Elektroden (2) verbindet, wobei das Verbindungselement aus einem Anschlussband aus elastischem Material besteht, das mit einem Leiter versehen ist, **dadurch gekennzeichnet, dass** das Verbindungselement (3) fähig ist, sich in das Steuerungsgehäuse (1) zurückzuschieben oder -zugleiten, wobei dennoch der elektrische Kontakt in dieser Steuerung (1) erhalten bleibt, so dass der Abstand zwischen den Elektroden (2) verändern werden kann.

2. Vorrichtung nach Anspruch 1, **dadurch gekennzeichnet, dass** jede Elektrode (2) am freien Ende des besagten Bandes kippbar (7) und austauschbar montiert wird.

3. Vorrichtung nach Anspruch 1 oder 2, **dadurch gekennzeichnet, dass** das besagte Anschlussband hinreichend elastisch ist, um sich einer beliebigen rundlichen Körperform anpassen zu können, gleichzeitig aber in Querrichtung ausreichend steif ist, um seine Position auf dem Körper zu halten.

4. Vorrichtung nach einem der Ansprüche 1 bis 3, **dadurch gekennzeichnet, dass** die Einzelsteuerung mit einem Ein- und Impulssequenawahlschalter (4) und mit Schaltern (5 und 6) zur Erhöung oder Senkung der Impulse ausgestattet ist.

5. Vorrichtung nach einem der Ansprüche 1 bis 4, **dadurch gekennzeichnet, dass** mehrere Einzelsteuerungen (1) vom Benutzer durch eine Master-Steuerung (10) gesteuert werden.

6. Vorrichtung nach Anspruch 5, **dadurch gekennzeichnet, dass** die Master-Steuerung (10) die notwendigen elektronischen Elemente für die Einstellung der Impulssequenzprogramme und/oder die individuelle Einstellung der Stromstärke umfasst und gleichzeitig Synchronisierungsfehler der Einzelsteuerungen (1) verhindert.

7. Vorrichtung nach einem der Ansprüche 5 oder 6, **dadurch gekennzeichnet, dass** die Master-Steuerung die Einzelsteuerungen (1) fernsteuert.

8. Vorrichtung nach einem der Ansprüche 5 oder 6, **dadurch gekennzeichnet, dass** die Master-Steuerung (10) auf der Haut angebracht wird und über die Haut Steuerungssignale an die Einzelsteuerungen (1) sendet.
